# EUROPÄISCHE PATENTANMELDUNG

(11) **EP 2 011 630 A1**
(43) Veröffentlichungstag der Anmeldung: **07.01.2009**
(21) Anmeldenummer: 07111622.2
(22) Anmeldetag: 03.07.2007
(51) Int. Cl.: B29C 59/16, B01L 3/00, G01N 33/52, G01N 33/48

(54) **Verfahren zur Herstellung eines Analyseelementes**

(71) Anmelder: F. Hoffmann-La Roche AG, 4070 Basel (CH); Roche Diagnostics GmbH, 68305 Mannheim (DE)
(72) Erfinder: Röper, Dr.Josef K., 67141 Neuhofen (DE); Finke, Dr.Werner, 64683 Einhausen (DE); Koschorrek, Beate, 69198 Schriesheim (DE)
(74) Vertreter: Hörschler, Wolfram Johannes

(57) **Zusammenfassung**

Die Erfindung bezieht sich auf ein Verfahren zur Herstellung eines Analyseelements, umfassend mindestens ein Testfeld (14) zur Analyse einer flüssigen Probe, wobei ein Träger (4) bereitgestellt wird, auf dem ein Polymergewebe (8) angeordnet ist. Zumindest ein Teilbereich (9) des Polymergewebes (8) wird mit UV-Laserlicht bestrahlt und dadurch hydrophobisiert.

## Beschreibung

Die Erfindung bezieht sich auf ein Analyseelement mit mindestens einem Testfeld zur Analyse einer flüssigen Probe und auf ein Verfahren zu dessen Herstellung.

Zur Analyse von Flüssigproben, beispielsweise Körperflüssigkeiten wie Blut oder Urin, werden häufig Analysegeräte verwendet, bei denen sich die zu analysierende Probe auf einem Testfeld eines Analyseelements befindet und in dem Testfeld gegebenenfalls mit einer oder mehreren Reagenzien reagiert, bevor sie analysiert wird. Die optische, insbesondere photometrische, und die elektrochemische Auswertung von Analyseelementen stellen die gebräuchlichsten Verfahren zur schnellen Bestimmung der Konzentration von Analyten in Proben dar. Analysesysteme mit Analyseelementen zur Probenanalyse werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Analyseelemente, die photometrisch oder elektrochemisch ausgewertet werden, einen großen Stellenwert.

Es gibt verschiedene Formen von Analyseelementen. Bekannt sind zum Beispiel im Wesentlichen quadratische Plättchen, die auch als Slides bezeichnet werden, in deren Mitte sich ein mehrschichtiges Testfeld befindet. Diagnostische Analyseelemente, die streifenförmig ausgebildet sind, werden als Teststreifen bezeichnet. Im Stand der Technik sind Analyseelemente umfassend beschrieben, beispielsweise in den Dokumenten CA 2311496 A1, US 5,846,837 A, US 6,036,919 A oder WO 97/02487.

Bei Kapillarspalt-Testelementen wird die Probenflüssigkeit von einem Probenaufgabeort zu einem davon entfernten Probendetektionsort mit Hilfe kapillarer Kräfte in einem Transportkanal (Kapillarkanal, Kapillarspalt) bewegt, um dort eine Nachweisreaktion einzugehen. Kapillarspalt-Testelemente sind beispielsweise aus CA 2549143 oder aus US 2003/0013147 A1 bekannt. Die Mikrokapillaren tragen eine Innenbeschichtung aus hydrophilen und gegebenenfalls aus hydrophoben Materialien. Durch hydrophile und hydrophobe Oberflächeneigenschaften der mit der Probenflüssigkeit in Kontakt tretenden Materialien kann der Flüssigkeitstransport gesteuert werden.

Weitere im Stand der Technik bekannte Analyseelemente sind Analysebänder mit einer Vielzahl von Testfeldern, die in einer Kassette aufgewickelt zur Verwendung in einem Analysegerät bereitgestellt werden. Solche Kassetten und Analysebänder werden zum Beispiel in den Dokumenten DE 103 32 488 A1, DE 103 43 896 A1, EP 1 424 040 A1, WO 2004/056269 A1 und CA 2506358 A1 beschrieben.

Die vorliegende Erfindung bezieht sich auf Analyseelemente beliebiger Form, insbesondere auf streifenförmige Testelemente (z.B. streifenförmige Kapillarspalt-Testelemente) und auf Analysebänder.

Analyseelemente weisen üblicherweise hydrophile und hydrophobe Bereiche auf. Die Begriffe "hydrophob" und "hydrophil" haben hierin die im Fachgebiet allgemein verstandenen Bedeutungen. Eine hydrophile Oberfläche weist eine gute Benetzbarkeit durch Wasser und eine hydrophobe Oberfläche eine schlechte Benetzbarkeit durch Wasser auf. Die Benetzbarkeit einer Oberfläche (und damit zum Beispiel die Fließgeschwindigkeit in einer mit dieser Oberfläche ausgestatteten Kapillare) lässt sich anhand des Kontaktwinkels α, den Wasser (bzw. eine Wasser enthaltende Probe) mit der Oberfläche bildet, ableiten. Bei der Berührung eines Flüssigkeitstropfens mit einer festen Unterlage können zwei Extremfälle auftreten:
- vollkommene Benetzung: die Adhäsionskräfte sind größer als die Kohäsionskräfte. Deshalb wird sich die Probe auf der Oberfläche des festen Körpers ausbreiten;
- unvollkommene Benetzung: die Adhäsionskräfte sind (wesentlich) kleiner als die Kohäsionskräfte. Deshalb wird sich die Flüssigkeit zu einem kugelförmigen Tropfen zusammenziehen.

Die Benetzbarkeit und folglich zum Beispiel die Fließgeschwindigkeit einer flüssigen Probe in einer Kapillare sind umso größer, je kleiner der Kontaktwinkel α ist. Die Füllzeit zur Füllung einer Kapillare pro Strecke steigt mit dem Kontaktwinkel exponentiell an. Bei Wasser enthaltenden Proben genügt die Angabe des Kontaktwinkels von Wasser, um die materialspezifischen kapillaren Eigenschaften zu charakterisieren. Der Begriff "hydrophobisieren" bedeutet in diesem Zusammenhang eine Veränderung einer Oberfläche, die eine Vergrößerung des Kontaktwinkels bewirkt, denen eine flüssige wasserhaltige Probe mit der Oberfläche bildet.

Im Stand der Technik werden hydrophile oder hydrophobe Oberflächeneigenschaften z.B. von Folien durch Imprägnierungs- und/oder Beschichtungsprozesse mit hierfür geeigneten Hilfsstoffen (z.B. Detergenzien oder Wachsen) erzeugt.

US 2001/0024805 A1 bezieht sich auf ein Verfahren zum Durchführen von Analysen, umfassend das Bereitstellen einer Vorrichtung zum Kultivieren von Mikroorganismen. Die Vorrichtung weist eine Auswertungsoberfläche auf, die hydrophile, flüssigkeitszurückhaltende Zonen und eine hydrophobe, erhöhte Fläche zwischen diesen Zonen aufweist. Die hydrophobe Oberfläche kann auf verschiedene Arten hydrophob gemacht werden. Zum Beispiel kann auf einem Polyethylenfilm, der durch Beimischen eines Netzmittels hydrophil gemacht wurde, eine dünne Schicht von acryliertem Silikon oder anderem hydrophoben Material aufgetragen werden.

WO 2005/054845 A1 betrifft ein analytisches Testelement zur Bestimmung eines Analyten in der Flüssigkeit. Das Testelement umfasst einen inerten Träger, eine Aufgabezone für Probenmaterial, eine Nachweiszone zur Bestimmung des Analyten und einen Kanal oder Spalt zum Transport von Flüssigkeit von der Aufgabezone zur Nachweiszone. Das Testelement weist zumindest in einem Bereich um die Aufgabezone eine hydrophob strukturierte Oberfläche auf. Die strukturierte hydrophobe Oberfläche mit Lotus-Effekt wird durch Beschichten, Tränken, Sprühen, Coexdrudieren oder Spritzguss erzeugt.

CA 2506358 A1 hat ein Verfahren zur Herstellung eines Analysebandes für Flüssigproben zum Gegenstand. Dabei wird ein aufrollbares Transportband mit einer Vielzahl von in Bandrichtung im Abstand voneinander befindlichen Testelementen versehen, die als selbstklebende Testetiketten auf dem Transportband angebracht werden. Die Testetiketten enthalten ein doppelseitiges Klebeband und als Testfeld einen Nachweisfilm mit geringer Breite zentriert auf der oberen Klebeschicht des Klebebandes, so dass seitliche Klebestreifen der Klebeschicht freigehalten werden. Darauf ist eine als Gewebe ausgebildete Decklage aufgebracht, die breiter als der Nachweisfilm ist und deren seitlich überstehende Ränder durch die seitlichen Klebestreifen fixiert werden. Die überstehenden Ränder der Decklage außerhalb des Nachweisfilms werden hydrophobisiert durch Bedrucken mit einer wasserabweisenden Imprägnierung, so dass nur eine zentrale Zone über dem Nachweisfilm eine flüssige Probe aufsaugen und zu dem Nachweisfilm transportieren kann.

Diese im Stand der Technik bekannten Verfahren zur Hydrophobisierung haben den Nachteil, dass zur Beschichtung verwendete Hilfsstoffe (z.B. Detergenzien, Thermotransferwachse) über Jahre mit gleich bleibender Qualität und mit zuverlässigen Lieferkonditionen zur Verfügung stehen müssen. Ferner können störende Wechselwirkungen bei gleichzeitiger Verwendung von hydrophilen und hydrophoben Reagenzien auftreten (z.B. Wechselwirkung eines aufgedruckten hydrophoben Thermotransferwachses mit einer Detergenzbeschichtung des bedruckten Gewebes). Außerdem können häufig keine scharfen Grenzen zwischen hydrophilen und hydrophob beschichteten Bereichen hergestellt werden.

Weiterhin wird im Stand der Technik die Bestrahlung mit elektromagnetischer Strahlung zur Hydrophobisierung eingesetzt. Gemäß EP 1291173 A1 wird eine hydrophile Schicht aus einer bestimmten wärmeempfindlichen Zusammensetzung hergestellt und durch IR-Strahlung belichtet, wodurch die belichteten Bereiche der Schicht stärker hydrophob werden. Gemäß WO 98/43739 A2 können durch Plasmabehandlungen hydrophil gemachte Oberflächen zurück in eine hydrophobe Oberfläche umgewandelt werden durch Anwendung von Lösungsmitteln, Ultraviolett-Licht oder Wärme. Diese Art der Umwandlung von hydrophilisierten Oberflächen in hydrophobe Oberflächen durch die in WO 98/43739 A2 beschriebenen unspezifischen Maßnahmen ist jedoch in der Praxis mit Nachteilen verbunden. So lässt sich beispielsweise feststellen, dass plasmabehandelte Oberflächen nur temporär ihren hochenergetischen, hydrophilen Zustand halten. Die Oberflächenenergien sind in der Regel erheblich erhöht und somit nicht langzeitstabil. Dies bedeutet jedoch, dass die Oberflächen nach einiger Zeit wieder in ihren hydrophoben Zustand übergehen, was zu einer erheblichen Veränderung der Testelementeigenschaften führen kann. Diese geringe Stabilität der Hydrophilisierung ist in der Regel auch dafür verantwortlich, dass das in WO 98/43739 A2 beschriebene Verfahren wirken kann, denn die offenbarten unspezifischen Prozesse und Einsatzstoffe wie Wärme, Lösungsmittel und UV-Licht würden bei einer stabil hydrophiliserten Oberfläche ansonsten mit hoher Wahrscheinlichkeit keine Überführung in einen hydrophoben Zustand herbeiführen.

Weiterhin sind die im Stand der Technik bekannten Verfahren aufwendig und kostenintensiv, da dem Hydrophobisieren vorbereitende Verfahren voraus gehen müssen (Beschichtung mit wärmeempfindlicher Zusammensetzung/Hydrophilisieren durch Plasmabehandlung).

Die Aufgabe der Erfindung besteht darin, die Nachteile des Standes der Technik zu vermeiden. Insbesondere ist es auch Aufgabe der Erfindung, ein Verfahren zur Herstellung eines Analyseelements bereit zu stellen, mit dem Oberflächenbereiche des Analyseelements kostengünstig und flexibel hydrophobisiert werden können.

Diese Aufgabe wird erfindungsgemäß gelöst durch ein Verfahren zur Herstellung eines Analyseelements mit mindestens einem Testfeld zur Analyse einer flüssigen Probe, wobei ein Träger bereitgestellt wird, auf dem ein Polymergewebe angeordnet ist. Zumindest ein Teilbereich des Polymergewebes wird mit UV-Laserlicht bestrahlt und dadurch hydrophobisiert.

Zur Herstellung des Analyseelements wird ein Träger bereitgestellt, auf dem ein Polymergewebe angeordnet ist. Der Träger kann beispielsweise eben-, insbesondere streifen- oder bandförmig, oder dreidimensional geformt sein.

Ein Testfeld ist in diesem Zusammenhang ein Feld, in dem eine flüssige Probe analysiert wird. Das Testfeld ist vorzugsweise auf dem Träger angeordnet. Beispielsweise ist ein Testfeld eine Nachweiszone, die so gestaltet ist, dass sich dort bestimmte Bestandteile der flüssigen Probe oder ihre Reaktion mit in der Nachweiszone vorhandenen Reagenzien nachweisen lassen. Ein Beispiel ist eine Zone, in der eine Nachweisreaktion für Glucose in einer flüssigen Probe (z.B. einer Blutprobe) und deren photometrische Auswertung stattfindet.

Das Polymergewebe im Sinne der Erfindung ist ein Gewebe aus Polymerfäden oder ein Vlies aus Polymerfasern. Das Polymergewebe besteht vorzugsweise aus einem Polymer ausgewählt aus der Gruppe bestehend aus Polyester, Polyamid, Polypropylen und Polyacrylnitril. Vorzugsweise ist das auf dem Träger angeordnete Polymergewebe (vor der Bestrahlung mit UV-Laserlicht) hydrophil oder hydrophilisiert und dient auf dem Analyseelement in (den nicht mit UV-Laserlicht bestrahlten) Teilbereichen zur Aufnahme und/oder zum Transport der flüssigen Probe. Besonders bevorzugt ist das Polymergewebe mit dem Testfeld direkt in Kontakt, z.B. bedeckt es das Testfeld ganz oder teilweise, so dass es in den nicht hydrophobisierten Bereichen eine flüssige Probe aufnehmen und an das Testfeld weiterleiten kann. Das Polymergewebe kann jedoch auch in mindestens einem Teilbereich selbst als Testfeld zur Analyse einer flüssigen Probe dienen und z.B. in diesem mindestens einen Teilbereich Reagenzien zum Nachweis eines Analyten in der flüssigen Probe enthalten. Das Polymergewebe kann ein Detergens zur Spreitung der Probe auf dem Gewebe aufweisen, enthält jedoch vorzugsweise selbst keine Reagenzien zum Nachweis eines Analyten. Diese Reagenzien befinden sich vorzugsweise lediglich in einem Nachweisfilm. Die flüssige Probe ist bevorzugt eine Wasser enthaltende Probe, z.B. Plasma, Blut, intestitielle Flüssigkeit, Urin, Speichel, Schweiß oder eine Probe der Wasseranalytik (insbesondere Altwasser).

Erfindungsgemäß wird zumindest ein Teilbereich des Polymergewebes mit UV-Laserlicht bestrahlt und dadurch in dem bestrahlten Bereich hydrophobisiert. UV-Laserlicht ist das von einem Laser emittierte Licht mit einer Wellenlänge im Bereich von 1 nm bis 380 nm. Bevorzugte Wellenlängen des für das erfindungsgemäße Verfahren verwendeten UV-Laserlichts sind 248 nm, 266 nm und 355 nm. Das UV-Laserlicht wird vorzugsweise durch diodengepumpte Festkörperlaser oder Excimer-Laser bereitgestellt. Vorzugsweise wird zumindest ein Teilbereich des Polymergewebes gezielt mit UV-Laserlicht bestrahlt. Gezielt bedeutete in diesem Zusammenhang, dass keine Masken oder ähnliches verwendet werden, sondern dass mindestens ein Laserstrahl mittels geeigneter optischer Komponenten auf den Teilbereich fokussiert wird und diesen Teilbereich abfährt (scannt), so dass eine ortsaufgelöste Hydrophobisierung des Polymergewebes erreicht wird. Alternativ oder zusätzlich kann jedoch auch ein Maskenverfahren bei der Bestrahlung mit Laserlicht eingesetzt werden, beispielsweise ein Masken-Belichtungsverfahren mit einem UV-Excimerlaser.

Im Gegensatz zu den aus dem Stand der Technik bekannten Verfahren, insbesondere dem in WO 98/43739 A2 offenbarten Verfahren, wird also durch Verwendung des UV-Laserlichtes, im Gegensatz zu unspezifischer Lichtbestrahlung, eine spezifische Behandlung durchgeführt. Durch diese spezifische Behandlung, welche ortsaufgelöst durchgeführt werden kann, können chemisch und langzeitstabile hydrophile Oberflächen mittels spezifischer UV-Laserstrahlung in einen neuen, hydrophoben Zustand überführt werden.

Der mittels UV-Laserlicht hydrophobisierte Teilbereich des Polymergewebes oder ein vollständig hydrophobisiertes Polymergewebe auf dem Analyseelement kann beispielsweise dazu dienen, die flüssige Probe in ihrem Fluss zu verlangsamen oder abzustoppen oder die Benetzung des Teilbereichs durch die flüssige Probe (z.B. bei der Probenaufgabe) zu verhindern. Durch das Hydrophobisieren eines bereitgestellten hydrophilen Polymergewebes in einem oder mehreren Teilbereichen kann ein Hydrophil-Hydrophob-Muster erzeugt werden.

Durch das Bestrahlen mit dem UV-Laserlicht wird das Polymergewebe in dem mit UV-Laserlicht bestrahlten Bereich strukturiert, d.h. die Oberflächenstrukturierung wird durch das Laserlicht verändert. Insbesondere kann die Polymeroberfläche des Polymergewebes durch das Bestrahlen mit Laserlicht aufgeraut werden. Zur Strukturierung wird vorzugsweise ein gepulster Laser verwendet, wobei die Polymeroberfläche in dem Teilbereich mit dem gepulsten Laserstrahl abgescannt wird und durch das Auftreffen der Laserpulse auf die Polymeroberfläche in einem gewissen Abstand zueinander die Polymeroberfläche strukturiert wird. Durch geeignete Wahl der Laserparameter (Wellenlänge, Leistung, Pulsrate etc.) können gezielt Mikrostrukturen erzeugt werden, die hydrophobe Eigenschaften hervorrufen. Durch das Laserlicht entstehen auf der Polymeroberfläche der Fäden oder Fasern des Polymergewebes verschmolzene, runde Strukturen (Erhebungen und Vertiefungen), deren mittlerer Abstand (z.B. von Vertiefung zu Vertiefung) mit dem Begriff "Hatch-Distance" bezeichnet wird.

Durch eine solche Strukturierung können zum Beispiel Teilbereiche der Polymeroberfläche des Polymergewebes so modifiziert werden, dass sie den so genannten "Lotus-Effekt" aufweisen. Dieser Effekt wird beispielsweise in WO 96/04123 A1, WO 00/58410 A1 oder WO 00/58415 A1 beschrieben. Eine solche Oberfläche weist Erhebungen und Vertiefungen auf, wobei der Abstand zwischen den Erhebungen im Bereich zwischen 0,1 bis 200 µm und die Höhe der Erhebungen im Bereich von 0,1 bis 100 µm liegt, und die Erhebungen hydrophob sind.

Ferner kann das Polymergewebe in dem Teilbereich durch das UV-Laserlicht so strukturiert werden, dass in die entstehenden Vertiefungen Fremdatome, vorzugsweise Luftmoleküle, eingelagert werden können, wodurch die Polymeroberfläche hydrophobisiert wird.

Das erfindungsgemäße Verfahren hat den Vorteil, dass bei der Bestrahlung mit UV-Laserlicht eine direkte Wechselwirkung zwischen dem Laserlicht und dem Polymergewebe stattfindet, die den gewünschten hydrophoben Effekt hervorruft. Zusätzliche Hilfsreagenzien sind nicht erforderlich. Aufwendige Fertigungsprozessschritte (Imprägnierungen, Tränkungen, Plasmabehandlungen) zur Vorbereitung der Hydrophobisierung entfallen. Die Bestrahlung mit UV-Laserlicht gewährt weitgehende Freiheit in der Gestaltung der Geometrie hydrophober Bereiche. Die UV-Laserbehandlung ist im Online-Betrieb gut einstell- und kontrollierbar.

Gemäß einer bevorzugten Ausführungsform der vorliegenden Erfindung ist das Polymergewebe ein monophiles Gewebe, das Fäden enthält, die weitgehend parallel oder senkrecht zueinander verlaufen, wobei die parallel zueinander verlaufenden Fäden einen Abstand zwischen 1 µm und 0,5 mm zueinander aufweisen, bevorzugt zwischen 0,1 und 200 µm. Ein monophiles Gewebe ist ein Gewebe, bei dem in Längs- und Querrichtung einzelne Fäden verwebt sind. Monophile Gewebe sind gegenüber herkömmlichen Gewebetypen definierter, z.B. hinsichtlich ihrer Porengröße, der Homogenität, der Luftdurchlässigkeit oder anderer Eigenschaften. Typische Maschenweiten können beispielsweise zwischen 105 und 285 Mikrometern liegen, typische Fadendurchmesser zwischen 42 und 145 Mikrometern, und typische Gewebedicken zwischen 63 und 260 Mikrometern.

Vorzugsweise wird für das erfindungsgemäße Herstellungsverfahren ein Träger bereitgestellt, auf dem das Polymergewebe mit einem Detergens beschichtet ist. Dabei ummantelt das Detergens vorzugsweise die einzelnen Fäden oder Fasern des Polymergewebes, so dass die Grenzflächenspannung zwischen der flüssigen Probe und der Oberfläche des Polymergewebes herabgesetzt wird. Das Detergens kann beispielsweise durch einen Tränkungsprozess auf das Polymergewebe aufgebracht werden.

Bevorzugt ist das Detergens mindestens ein Detergens ausgewählt aus der Gruppe bestehend aus Natriumdioctylsulfosuccinat (DONS), Mega-8® (Ocatanoyl-N-methylglucamid) und Nonylphenolethoxylaten, insbesondere Polyethylenglycol-[4-(1,1,3,3-tetramethylbutyl)phenyl]-ether (Triton®). Das Detergens, insbesondere das DONS, kann beispielsweise auf ein Polyestergewebe durch Tränken des Gewebes mit in Ethanol gelöstem DONS und anschließendes Trocknen als feste Schicht aufgebracht werden.

Gemäß einer bevorzugten Ausführungsform der vorliegenden Erfindung wird das Detergens in dem mit UV-Laserlicht bestrahlten Teilbereich des Polymergewebes zumindest teilweise durch das UV-Laserlicht entfernt. Die durch UV-Laserbestrahlung hervorgerufene Hydrophobisierung beruht dabei auf einer Entfernung des Detergens von dem Polymergewebe, insbesondere durch Ablation, und gegebenenfalls zusätzlich auf der Strukturierung der Oberfläche des Polymergewebes.

Die gezielte UV-Laserbestrahlung eines mit Detergens beschichteten Polymergewebes hat eine Vielzahl von Vorteilen, z.B. im Vergleich zu der im Stand der Technik bekannten Bedruckung eines solchen mit Detergens beschichteten Polymergewebes mit einer hydrophoben Substanz (insbesondere die Bedruckung eines mit DONS imprägnierten PET-Gewebes mit Thermotransferwachs). Beim bekannten Thermotransferwachsauftrag wird auf einer Folie aufgebrachtes Wachsgemisch, durch Wärme verflüssigt und auf das mit DONS imprägnierte PET-Gewebe aufgedruckt. Hierbei findet eine Vermischung des hydrophilen DONS mit dem hydrophoben Wachsgemisch statt. Voraussetzung für die Erzeugung einer funktionierenden Hydrophobsperre ist hierbei eine genaue Einstellung des Mischungsverhältnisses aus Detergens (DONS) und Wachs. Die Herstellung definierter Grenzen zwischen hydrophobisierten Bereichen mit Wachs und hydrophilen Bereichen ohne Wachs ist nicht möglich, sondern es entstehen Übergangsbereiche. Durch die UV-Laserbestrahlung werden diese Nachteile vermieden. Eine Wechselwirkung von hydrophilen und hydrophoben Reagenzien findet nicht statt. Für die Gestaltung von hydrophilen und hydrophoben Bereichen auf Analyseelementen ergibt sich eine Vielzahl von Möglichkeiten. Weiterhin ergibt sich über einen Zeitraum von mindestens 6 Monaten eine hohe Stabilität der durch die UV-Laserbehandlung erzeugten hydrophoben Eigenschaft.

Eine bevorzugte Ausführungsvariante des erfindungsgemäßen Verfahrens besteht darin, dass das Analyseelement ein Testelement zur Bestimmung eines Analyten in einer flüssigen Probe ist, das eine Aufgabezone für die flüssige Probe umfasst, wobei das Polymergewebe in einem Bereich um die Aufgabezone mit UV-Laserlicht bestrahlt und dadurch hydrophobisiert wird. Das Testelement ist dabei vorzugsweise ein Teststreifen oder ein auf einem Analyseband angeordnetes Testetikett.

Eine Aufgabezone ist in diesem Zusammenhang ein Bereich des Analyseelements, der dazu vorgesehen ist, eine flüssige Probe aufzunehmen, die auf dem Analyseelement transportiert, gemischt, getrennt, mit Reagenzien kontaktiert und/oder anderweitig prozessiert und analysiert wird.

Durch das Hydrophobisieren im Bereich um die Aufgabezone, in der sich zum Beispiel die Öffnung eines Kapillarkanals oder ein hydrophiles Polymergewebe befindet, wird die Aufgabezone klar begrenzt. Beim Auftragen der flüssigen Probe (z.B. von Blut) auf die Aufgabezone wird überschüssige Probenflüssigkeit entweder durch die Aufgabezone aufgenommen (z.B. in einen Kapillarkanal eingesaugt oder durch ein hydrophiles Polymergewebe zu einer Analysezone weiter transportiert) oder tropft von dem hydrophobisierten Bereich ab, so dass nur die Aufgabezone benetzt wird und eine Verschmutzung der Umgebung der Aufgabezone des Analyseelements und eines das Analyseelement aufnehmenden Messgeräts vermieden wird.

Gemäß einer bevorzugten Ausführungsform der vorliegenden Erfindung wird zur Herstellung eines Testelements ein Nachweisfilm unter Freihaltung von Rändern auf den Träger aufgebracht, der Nachweisfilm von dem Polymergewebe überdeckt, wobei das Polymergewebe in seitlichen Bereichen über den Nachweisfilm übersteht und die freien Ränder des Trägers bedeckt, und das Polymergewebe durch Bestrahlen mit UV-Laserlicht in den seitlichen Bereichen hydrophobisiert. Bevorzugt ist der dabei verwendete Träger ein Klebeband, insbesondere ein doppelseitiges Klebeband, mit dem das Testelement als selbstklebendes Testetikett auf ein Transportband übertragen wird. Der Aufbau des Analyseelements entspricht dabei vorzugsweise dem in CA 2506358 A1 beschriebenen Analyseband, mit dem Unterschied, dass das Polymergewebe außerhalb des Nachweisfilms nicht mit einer wasserabweisenden Imprägnierung bedruckt wird, sondern durch Bestrahlen mit UV-Laserlicht in den seitlichen Bereichen hydrophobisiert wird. Auf die CA 2506358 A1 wird daher ausdrücklich Bezug genommen.

Die Erfindung bezieht sich weiterhin auf ein Analyseelement, das nach dem erfindungsgemäßen Verfahren hergestellt wurde und das mindestens ein Testfeld zur Analyse einer flüssigen Probe umfasst, wobei das Analyseelement einen Träger enthält, auf dem ein Polymergewebe angeordnet ist. Zumindest ein Teilbereich des Polymergewebes ist durch Bestrahlung mit UV-Laserlicht hydrophobisiert.

Gemäß einer bevorzugten Ausführungsvariante ist das erfindungsgemäße Analyseelement ein Analyseband mit einer Vielzahl von in Bandrichtung voneinander beabstandeten Testelementen.

Anhand der Zeichnung wird die Erfindung nachstehend näher erläutert.

Es zeigt
- Figur 1: schematisch eine perspektivische Ansicht eines Analyseelements gemäß dem Stand der Technik in Form eines Analysebandes,
- Figur 2: schematisch eine perspektivische Ansicht eines erfindungsgemäßen Analyseelements in Form eines Analysebandes, hergestellt nach dem erfindungsgemäßen Verfahren und
- Figuren 3A und 3B: und eine vergrößerte Ansicht eines durch UV-Laserbestrahlung hydrophobisierten Polymergewebes vor der Laserbehandlung (Figur 3A) nach der Laserbehandlung (Figur 3B).

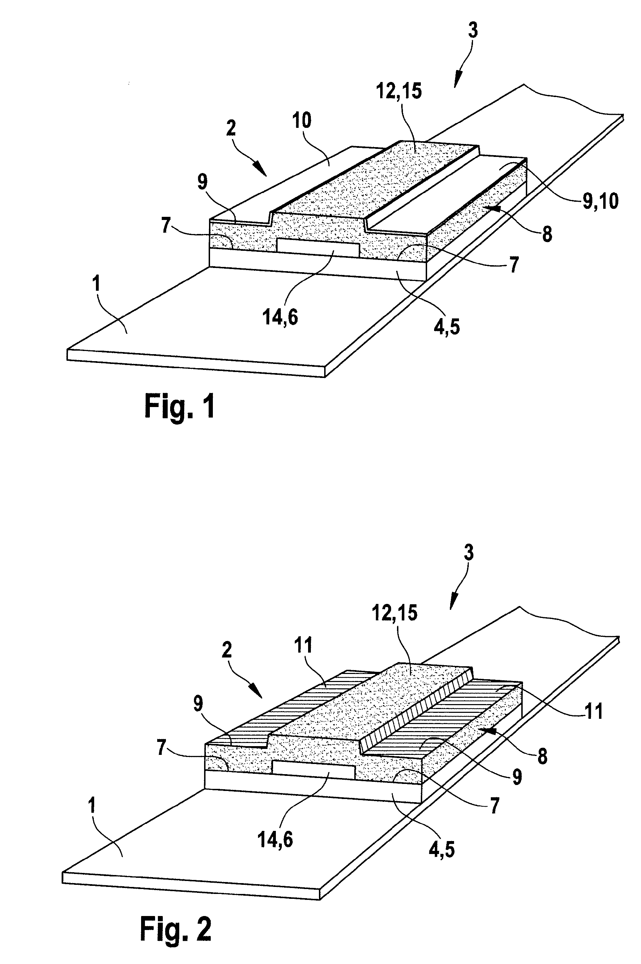

Figur 1 zeigt ein im Stand der Technik bekanntes Analyseelement. Das Analyseelement ist ein in Figur 1 ausschnittsweise dargestelltes Analyseband 3, das ein aufrollbares Transportband 1 und eine Vielzahl von darauf angeordneten, in Bandrichtung voneinander beabstandeten Testelementen 2 umfasst. Die Testelemente 2 (von denen nur eines in Figur 1 dargestellt ist) sind zum Beispiel zur Analyse von Körperflüssigkeiten, insbesondere von Blut, vorgesehen.

Das Testelement 2 ist mehrlagig als selbstklebendes Testetikett aufgebaut. Als Träger 4 des Testelements 2 dient ein doppelseitiges Klebeband 5. Auf der oberen Klebeschicht des doppelseitigen Klebebandes 5 ist ein Nachweisfilm 6 mit geringerer Breite zentriert aufgeklebt, so dass seitliche Ränder 7 auf dem Träger freigehalten werden. Der Nachweisfilm 6 wird von einem Polymergewebe 8 überdeckt. Das Polymergewebe 8 ist breiter als der Nachweisfilm 6, so dass das Polymergewebe 8 in seitlichen Bereichen 9 über den Nachweisfilm 6 übersteht. In den seitlichen Bereichen 9 wird das Polymergewebe 8 daher durch die Ränder 7 des Klebebandes 5 fixiert.

Im Stand der Technik sind die seitlichen Bereiche 9 mit hydrophoben Thermotransferwachs 10 als wasserabweisende Imprägnierung bedruckt, so dass nur das zentrale Testfeld (Nachweiszone 14) die aufzubringende flüssige Probe aufsaugen und begrenzt ausbreiten kann. Das Polymergewebe 8 enthält ein Detergens, das sich bei der Imprägnierung mit Thermotransferwachs 10 mit diesem vermischt. Dabei muss eine kritische Balance zwischen Thermotransferwachs 10 und Detergens eingehalten werden, um die gewünschte Hydrophobizität der seitlichen Bereiche 9 einzustellen.

Figur 2 zeigt ein erfindungsgemäßes Analyseelement in Form eines Analysebandes, hergestellt nach dem erfindungsgemäßen Verfahren.

Das Analyseelement ist im Wesentlichen wie das Analyseelement gemäß Figur 1 aufgebaut. Gleiche Bezugszeichen bezeichnen gleiche Bestandteile dieses Analysebandes 3. Im Unterschied zu dem Analyseelement gemäß Figur 1 weist das erfindungsgemäße Analyseelement gemäß Figur 2 jedoch keine Bedruckung aus Thermotransferwachs auf. Stattdessen sind die seitlichen Bereiche 9 des mit Detergens imprägnierten Polymergewebes 8 durch Bestrahlen mit UV-Laserlicht hydrophobisiert. Die Funktion dieser beidseitig angeordneten hydrophoben Bereiche 11 ist es, eine lokalisierte Probenauftragung auf das als Aufgabezone 15 vorgesehene mittig angeordnete Polymergewebe 12 zu ermöglichen, das hydrophil ausgebildet ist, ohne eine Kontamination der Umgebung mit der flüssigen Probe, die die seitlichen hydrophoben Bereiche 11 nicht oder nur schlecht benetzt.

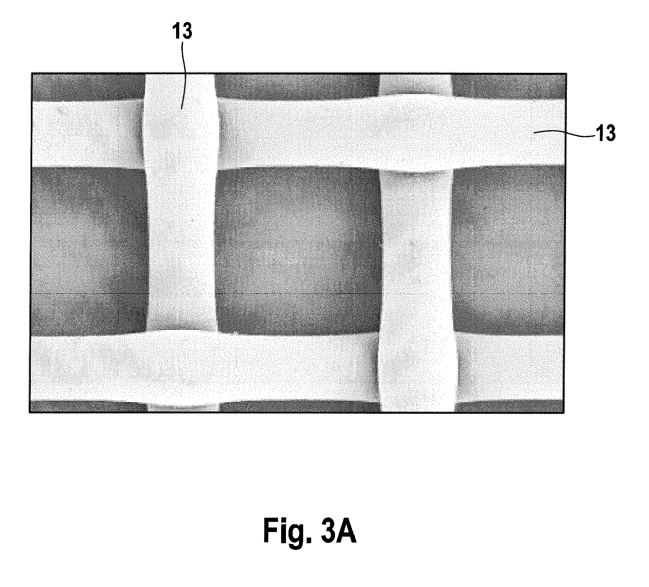

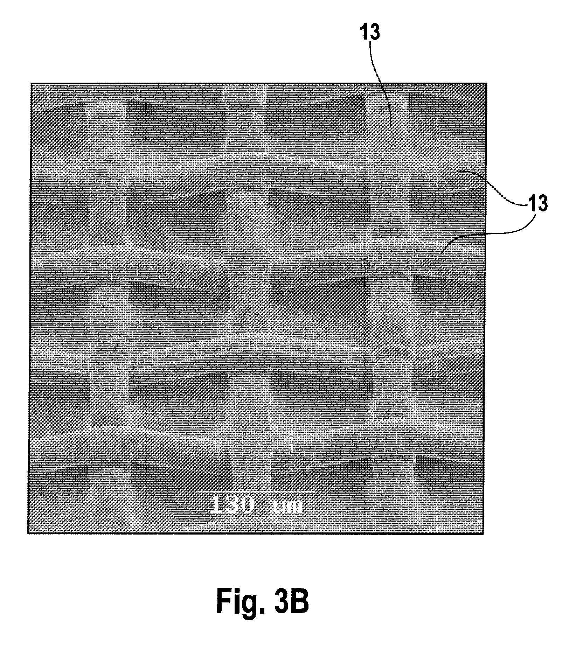

In den Figuren 3A und 3B ist eine vergrößerte Ansicht eines durch UV-Laserbestrahlung hydrophobisierten Polymergewebes dargestellt. Dabei zeigt die Figur 3A ein Polymergewebe vor der Laserbehandlung, wohingegen Figur 3B das Gewebe nach der Laserbehandlung zeigt. Im Vergleich der beiden Bilder ist deutlich zu erkennen, dass die Laserbehandlung eine Oberflächenrauhigkeit erzeugt, welche sich auf die Benetzungseigenschaften der Oberflächen auswirkt.

Das Polymergewebe ist ein Polyestergewebe. Es ist ein monophiles Gewebe, das Fäden 13 enthält, die weitgehend parallel oder senkrecht zueinander verlaufen, wobei die parallel zueinander verlaufenden Fäden 13 einen Abstand von ca. 80-120 µm

Die Laserbestrahlung erfolgte unter Einsatz verschiedener UV-Lasertypen. So wurde ein eines Excimer-Laser mit einer Wellenlänge von 248 nm, einer Frequenz von 100 Hz, einer Pulsenergie von 7 mJ und einer Spot Size von 400 Mikrometern eingesetzt. Die Anzahl der Pulse wurde in drei Experimenten variiert zwischen 10 Pulsen, 15 Pulsen und 20 Pulsen. Weiterhin wurde ein 4-f Diodenlaser mit einer Wellenlänge von 266 nm eingesetzt. Der Diodenlaser wurde ebenfalls gepulst betrieben, mit einer Pulsfrequenz von 30 kHz und einer Pulsenergie von 10 Mikrojoule. Die Spot Size betrug 18 Mikrometer.

Das Polymergewebe wurde in diesem Fall gemäß der obigen Beschreibung mit DONS imprägniert. Die Laserbehandlung erzeugte eine feine Strukturierung an den Gewebefäden, verbunden mit einer lokalen Entfernung des DONS. Diese Strukturierung ist in Figur 3B deutlich in Form einer Riffelung der Fäden 13 zu erkennen. Dabei erfolgte eine Kontaktwinkelveränderung hin in Richtung zum superhydrophoben Bereich, also in einen Bereich mit einem stehenden Tropfen.

### Bezugszeichenliste

- 1.: Transportband
- 2.: Testelement
- 3.: Analyseband
- 4.: Träger
- 5.: doppelseitiges Klebeband
- 6.: Nachweisfilm
- 7.: Ränder
- 8.: Polymergewebe
- 9.: seitliche Bereiche
- 10.: Thermotransferwachs
- 11.: Hydrophobe Bereiche
- 12.: mittig angeordnetes Polymergewebe
- 13.: Fäden
- 14.: Testfeld/Nachweiszone
- 15.: Aufgabezone

## Patentansprüche

1. Verfahren zur Herstellung eines Analyseelements mit mindestens einem Testfeld (14) zur Analyse einer flüssigen Probe, wobei ein Träger (4) bereitgestellt wird, auf dem ein Polymergewebe (8) angeordnet ist, **dadurch gekennzeichnet, dass** zumindest ein Teilbereich (9) des Polymergewebes (8) mit UV-Laserlicht bestrahlt und **dadurch** hydrophobisiert wird.

2. Verfahren gemäß Anspruch 1, **dadurch gekennzeichnet, dass** das Polymergewebe (8) ein monophiles Gewebe ist, das Fäden (13) enthält, die weitgehend parallel oder senkrecht zueinander verlaufen, wobei die parallel zueinander verlaufenden Fäden (13) einen Abstand zwischen 1 µm und 0,5 mm zueinander aufweisen.

3. Verfahren gemäß einem der Ansprüche 1 oder 2, **dadurch gekennzeichnet, dass** ein Träger bereitgestellt wird, auf dem das Polymergewebe (8) mit einem Detergens beschichtet ist.

4. Verfahren gemäß Anspruch 3, **dadurch gekennzeichnet, dass** das Detergens mindestens ein Detergens ausgewählt aus der Gruppe bestehend aus Natriumdioctylsulfosuccinat (DONS), Ocatanoyl-N-methylglucamid und Nonylphenolethoxylaten, insbesondere Polyethylenglycol-[4-(1,1,3,3-tetramethylbutyl)phenyl]-ether.

5. Verfahren gemäß einem der Ansprüche 3 oder 4, **dadurch gekennzeichnet, dass** das Detergens in dem mit UV-Laserlicht bestrahlten Teilbereich (9, 11) des Polymergewebes zumindest teilweise durch das UV-Laserlicht entfernt wird.

6. Verfahren gemäß einem der Ansprüche 1 bis 5, **dadurch gekennzeichnet, dass** das Analyseelement ein Testelement (2) zur Bestimmung eines Analyten in einer flüssigen Probe ist, das einer Aufgabezone (15) für die flüssige Probe umfasst, wobei das Polymergewebe (8) in einem Bereich (9) um die Aufgabezone (15) mit UV-Laserlicht bestrahlt und **dadurch** hydrophobisiert wird.

7. Verfahren gemäß einem der Ansprüche 1 bis 6, **dadurch gekennzeichnet, dass** zur Herstellung eines Testelements (2) ein Nachweisfilm (6) unter Freihaltung von Rändern (7) auf den Träger (4) aufgebracht wird, der Nachweisfilm (6) von dem Polymergewebe (8) überdeckt wird, wobei das Polymergewebe (8) in seitlichen Bereichen (9) über den Nachweisfilm (6) übersteht und die freien Ränder (7) des Trägers (4) bedeckt, und das Polymergewebe (8) durch Bestrahlung mit UV-Laserlicht in den seitlichen Bereichen (9) hydrophobisiert wird.

8. Verfahren gemäß Anspruch 7, **dadurch gekennzeichnet, dass** der Träger (4) ein Klebeband (5) ist, mit dem das Testelement (2) als selbstklebendes Testetikett auf ein Transportband (1) übertragen wird.

9. Analyseelement, hergestellt nach einem Verfahren gemäß einem der Ansprüche 1 bis 8, umfassend mindestens ein Testfeld (14) zur Analyse einer flüssigen Probe, wobei das Analyseelement einen Träger (4) enthält, auf dem ein Polymergewebe (8) angeordnet ist, **dadurch gekennzeichnet, dass** zumindest ein Teilbereich (9, 11) des Polymergewebes (8) durch Bestrahlung mit UV-Laserlicht hydrophobisiert ist.

10. Analyseelement gemäß Anspruch 9, **dadurch gekennzeichnet, dass** das Analyseelement ein Analyseband (3) mit einer Vielzahl von in Bandrichtung voneinander beabstandeten Testelementen (2) ist.
